# EUROPEAN PATENT APPLICATION

(11) **EP 4 691 380 A1**
(43) Date of publication of application: **11.02.2026**
(21) Application number: 24777799.8
(22) Date of filing: 19.03.2024
(51) Int. Cl.: A61B 17/072, A61B 90/00

(54) **ELECTRIC STAPLER**

(30) Priority: 27.03.2023 CN 202310310801
(71) Applicant: B. J. Zh. F. Panther Medical Equipment Co., Ltd., Beijing 102209 (CN)
(72) Inventor: LIU, Qing, Beijing 102209 (CN); CHEN, Xiaoqiang, Beijing 102209 (CN); YANG, Xuelan, Beijing 102209 (CN); LIU, Libo, Beijing 102209 (CN)
(74) Representative: Huang, Liwei
(86) International application number: PCT/CN2024/082458
(87) International publication number: WO 2024/199010

(57) **Abstract**

An electric stapler comprises a main drive assembly, a rotating assembly and a swing assembly; the main drive assembly is used for driving an actuator to perform closing, stapling and retracting, the rotating assembly is used for driving the actuator to rotate 360 degrees, the swing assembly is used for driving the actuator to swing left and right; wherein, all the main drive assembly, the rotating assembly and the swing assembly comprise a motor and a gear assembly; the main drive assembly, the rotating assembly and the swing assembly can independently operate, and do not affect each other during operation. The main drive assembly, the rotating assembly and the swing assembly are configured to be electric assemblies, thereby improving the surgical efficiency, reducing the operation difficulty of doctors.

## Description

### Technical Field

The present invention relates to the field of medical devices, and in particular to an electric stapler.

### Background

Endoscopic staplers are commonly used devices in surgical operations, which can help doctors quickly complete the action of cutting and suturing tissues. At present, there are two main types of endoscopic staplers: one is a manual endoscopic stapler, which is manually controlled the cartridge assembly firing to suture tissues and other operations. The firing handle needs to be manually gripped multiple times during use, so that the mechanical transmission mechanism of the stapler pushes the cartridge assembly to close and form. The manual endoscopic stapler requires certain requirements for the doctor's actual operating skills. Improper operation will cause pulling and tearing of the sutured tissues. The other is an electric endoscopic stapler, which is partially operated in an electric form, allowing the doctor to perform partial operation of the endoscopic stapler by touching the buttons on the endoscopic fixed handle with his fingers. With the development of technology, electric staplers in some operations have been widely used. However, since the operation of the stapler includes the swing, rotation of the actuator, and the closing and firing of the stapler cartridge, etc. The electric stapler in the prior art is not intelligent enough, and the technical solution that all the above three operations are driven by motors has not been realized, resulting in the doctor's operation not being fully intelligent.

### Summary of the Invention

The present invention aims to solve the technical problem in the prior art that the electric stapler cannot be operated intelligently.

In order to solve the above technical problem, the technical solutions provided by the present invention are as follows:

An electric stapler comprises a main drive assembly, a rotating assembly and a swing assembly; the main drive assembly is used to drive an actuator to close, staple and retract, the rotating assembly is used to drive the actuator to rotate 360 degrees, the swing assembly is used to drive the actuator to swing left and right; wherein all the main drive assembly, the rotating assembly and the swing assembly comprise a motor and a gear assembly; the main drive assembly, the rotating assembly and the swing assembly can be operated independently and do not affect each other when performing operations.

Further, the main drive assembly includes: a firing motor, a firing gear, a firing driven gear, a firing rack, a distal end of the firing rack is connected to a proximal end of a firing push rod, the firing push rod can rotate freely around the axis relative to the firing rack; the firing gear is engaged with the firing driven gear, the firing driven gear is engaged with the firing rack, when the firing motor rotates forward or reversely according to a main control instruction, a rotational motion is converted into a linear motion through the firing gear, the firing driven gear, the firing rack, thereby driving a distal actuator to perform closing, stapling and retracting functions.

Further, the rotating assembly includes a rotating motor, a rotating drive gear, a rotating driven gear, the rotating assembly is connected to a barrel assembly through a fixed block, wherein, the rotating drive gear is engaged with the rotating driven gear, the rotating driven gear includes a gear portion and a cylindrical portion, the gear portion includes a center circular hole, and one end of the cylindrical portion passes through the center circular hole and is fixed to the gear portion.

Further, there are two fixed blocks, which are clamped together by a clamping assembly, each fixed block is respectively provided with a first clamping platform and a second clamping platform, the cylindrical portion is provided with a cylindrical portion clamping groove corresponding to the second clamping platform, the fixed block is fixed to the rotating driven gear through the second clamping platform and the cylindrical portion clamping groove, the fixed block is fixed to the barrel assembly through the first clamping platform and the barrel assembly clamping groove; when the rotating driven gear rotates, the barrel assembly is driven to rotate through the fixed block, thereby realizing a rotation function of the distal actuator.

Further, the swing assembly includes a swing motor, a swing screw, a swing drive gear and a swing driven gear, the swing drive gear and the swing driven gear are engaged, the swing driven gear and the swing screw are spirally matched, the swing motor rotates to drive the swing screw to move back and forth through the swing drive gear and the swing driven gear; the swing screw passes through the cylindrical portion of the rotating driven gear and can move back and forth in the cylindrical portion; the swing screw is a hollow structure, and the firing push rod passes through the swing screw and can move back and forth in the swing screw.

Further, the swing driven gear is provided with a center hole, a wall of the center hole is provided with threads, an outer wall of the swing screw is correspondingly provided with threads.

Further, the swing assembly also includes a swing crank and a swing connecting rod, and there is a crank ring groove on the swing screw, which cooperates with an annular clamping platform on the swing crank, and the swing crank is fixedly connected to the swing connecting rod through a crank pull pin. When the swing screw moves back and forth, it drives the swing crank to move back and forth, thereby driving the swing connecting rod to move back and forth, so as to realize the left and right swing function of the distal actuator.

Further, the electric stapler also includes a first bracket, a second bracket, a gear rack, the second bracket and the gear rack are fixed on the first bracket, the main drive assembly is fixed on the first bracket, the rotating motor and the swing motor are fixed on the second bracket, the gear assemblies of the rotating assembly and the swing assembly are arranged in the gear rack.

Further, the electric stapler also includes a main circuit board and a sub-circuit board, the main circuit board and the sub-circuit board are respectively fixed on both sides of the first bracket, the main circuit board is provided with a control circuit and a first guide button of the electric stapler, and the sub-circuit board is provided with a second guide button, wherein, the first guide button and the second guide button are symmetrically arranged and have the same functions, the first guide button and the second guide button have trigger functions in five directions, namely, left and right, front and back, and downward pressing, which respectively correspond to the rotation of the actuator, the left and right swing of the actuator, and the start-firing confirmation function of the actuator.

After adopting such a design, the present invention has at least the following advantages:
(1) The electric stapler of the present invention can facilitate the doctor's operation by providing an electric main drive assembly, a rotating assembly and a swing assembly, thereby improving efficiency, shortening the operation time.
(2) The electric stapler of the present invention is operated by buttons, which can be operated with one hand, greatly reducing the difficulty of operation.
(3) The present invention has an ingenious structure, good effect, low cost and easy process implementation.

### Description of the Accompanying Drawings

FIG 1 is a schematic diagram of the overall structure of an electric stapler of the present invention;
FIG 2 is a schematic diagram of the internal structure of a drive portion of the electric stapler of the present invention;
FIG 3 is a schematic diagram of the structure of a swing assembly and a rotating assembly of the present invention;
FIG 4 is a schematic diagram of the internal structure of the swing assembly and the rotating assembly of the present invention;
FIG 5 is a schematic diagram of the structure of a first circuit board installation structure of the present invention;
FIG 6 is a schematic diagram of the structure of a second circuit board installation structure of the present invention;
FIG 7 is a schematic diagram of the structure of a fixed block of the present invention;
FIG 8 is a schematic diagram of the structure of a rotating screw of the present invention;
FIG 9 is a schematic diagram of the structure of a swing crank of the present invention.

Reference numerals, 101 a firing motor, 102 a firing gear, 103 a firing driven gear, 104 a firing rack, 105 a first bracket, 106 a main circuit board, 1061 a first guide button, 107 a sub-circuit board, 1071 a second guide button, 200 a second bracket, 201 a rotating motor, 202 a swing motor, 203 a gear rack, 2031 a rotating driven gear, 20310 a cylindrical portion clamping groove, 2032 a rotating drive gear, 2033 a swing drive gear, 2034 a swing driven gear, 2041 a fixed block, 20410 a first clamping platform, 20411 a second clamping platform, 205 a swing screw, 2052 a crank ring groove, 209 a swing crank, 2091 an annular clamping platform, 210 a crank pull pin, 211 a swing connecting rod, 30 a barrel assembly, 301 a firing push rod, 3020 a barrel assembly groove.

### Embodiments of the Invention

The following will be combined with the drawings in the embodiments of the present invention to clearly and completely describe the technical solutions in the embodiments of the present invention. Obviously, the described embodiments are only part of the embodiments of the present invention, not all of the embodiments. Based on the embodiments of the present invention, all other embodiments obtained by ordinary technicians in this field without creative work are within the scope of protection of the present invention.

In this document, "upper", "lower", etc. are only used to indicate the relative position relationship between related parts, rather than to limit the absolute positions of these related parts.

In the following, the end of each component close to the operator is defined as the proximal end, and the end away from the operator is defined as the distal end. The expressions of the proximal end and the distal end are only used to make the description simple and clear, and should not be understood as any limitation to the present invention.

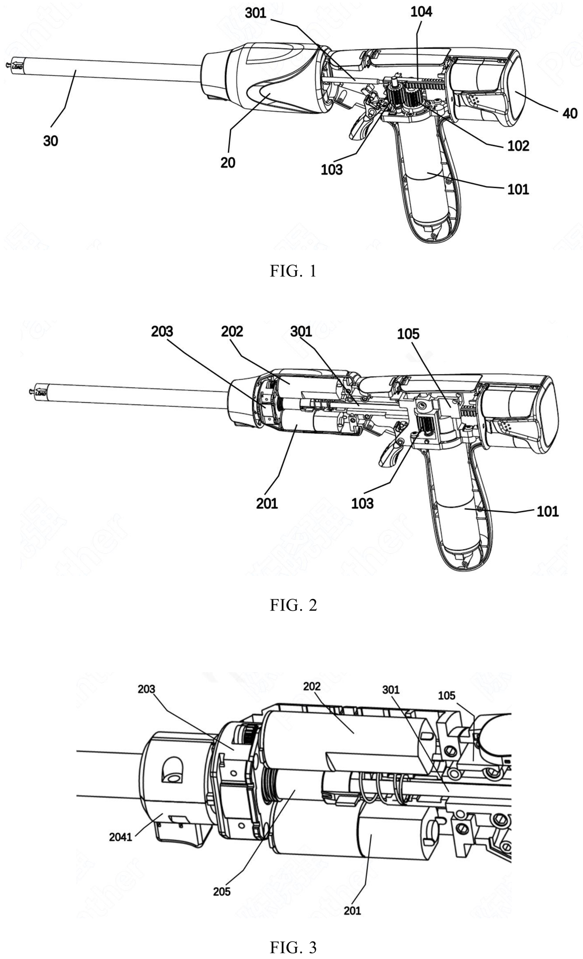

Referring to FIGs 1-9, an electric stapler comprises a main drive assembly, a rotating assembly and a swing assembly; the main drive assembly is used to drive an actuator to close, staple and retract, the rotating assembly is used to drive the actuator to rotate 360 degrees, the swing assembly is used to drive the actuator to swing left and right; wherein all the main drive assembly, the rotating assembly and the swing assembly comprise a motor and a gear assembly; the main drive assembly, the rotating assembly and the swing assembly can be operated independently and do not affect each other when performing operations.

Referring to FIGs 1-2, the main drive assembly includes: a firing motor 101, a firing gear 102, a firing driven gear 103, a firing rack 104, a distal end of the firing rack 104 is connected to a proximal end of a firing push rod 301, and the firing push rod 301 can rotate freely around the axis relative to the firing rack 104; the firing gear 102 is engaged with the firing driven gear 103, the firing driven gear 103 is engaged with the firing rack 104, when the firing motor 101 rotates forward or reversely according to a main control instruction, a rotational motion is converted into a linear motion through the firing gear 102, the firing driven gear 103, the firing rack 104, thereby driving a distal actuator to perform closing, stapling and retracting functions.

Referring to FIGs 2-4, the rotating assembly includes a rotating motor 201, a rotating drive gear 2032, a rotating driven gear 2031, the rotating assembly is connected to a barrel assembly 30 through a fixed block 2041, wherein, the rotating driven gear 2031 includes a gear portion and a cylindrical portion, the gear portion includes a center circular hole, and one end of the cylindrical portion passes through the center circular hole and is fixed to the gear portion. The rotating drive gear 2032 is engaged with the rotating driven gear 2031, the rotating motor 201 includes a non-circular output shaft, the rotating drive gear 2032 includes a center hole matching the output shaft shape, the output shaft passes through the center hole of the rotating drive gear 2032, driving the rotating drive gear 2032 to rotate around the axis, the rotating drive gear 2032 rotates to drive the rotating driven gear 2031 to rotate, the barrel assembly 30 is driven to rotate through the fixed block 2041.

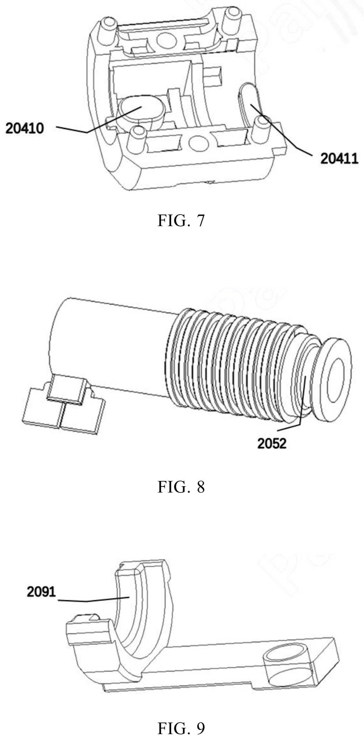

Further, there are two fixed blocks 2041, which are clamped together by a clamping assembly. The specific structure of the clamping assembly is not limited, as long as the two fixed blocks 2041 can be clamped together. Preferably, referring to FIG 7, the clamping structure includes an embossment provided on one fixed block 2041 and a groove provided on the other fixed block 2041 corresponding to the embossment. Each fixed block 2041 is provided with a first clamping platform 20410 and a second clamping platform 20411, the cylindrical portion is provided with a cylindrical portion clamping groove 20310 corresponding to the second clamping platform 20411, the fixed block 2041 is fixed to the rotating driven gear 2031 through the second clamping platform 20411 and the cylindrical portion clamping groove 20310, the fixed block 2041 is fixed to the barrel assembly 30 through the first clamping platform 20410 and a barrel assembly clamping groove 3020. When the rotating driven gear 2031 rotates, the barrel assembly 30 and the cartridge assembly loaded thereon are driven to rotate through the fixed block 2041, thereby realizing a rotation function of the distal actuator.

The swing assembly includes a swing motor 202, a swing screw 205, a swing drive gear 2033 and a swing driven gear 2034, the swing drive gear 2033 and the swing driven gear 2034 are engaged, the swing driven gear 2034 and the swing screw 205 are spirally matched, the swing motor 202 rotates to drive the swing screw to move back and forth through the swing drive gear 2033 and the swing driven gear 2034; the swing screw 205 passes through the cylindrical portion of the rotating driven gear 2031 and can move back and forth in the cylindrical portion; the swing screw 205 is a hollow structure, and the firing push rod 301 passes through the swing screw 205 and can move back and forth in the swing screw 205.

Further, the swing driven gear 2034 is provided with a center hole, a wall of the center hole is provided with threads, an outer wall of the swing screw 205 is correspondingly provided with threads; preferably, threads can be triangular threads, trapezoidal threads or rectangular threads. The swing motor 202 includes a non-circular output shaft, the swing drive gear 2033 is provided with a center hole matching the shape of the output shaft, the output shaft passes through the center hole of the swing drive gear 2033 to drive the swing drive gear 2033 to rotate around the axis, the rotation of the swing drive gear 2033 drives the swing driven gear 2034 to rotate, the rotation of the swing drive gear 2034 drives the swing screw 205 to move back and forth.

Further, the swing assembly also includes a swing crank 209 and a swing connecting rod 211, and there is a crank ring groove 2052 (Referring to FIG 8) on the swing screw 205, which cooperates with an annular clamping platform 2091 on the swing crank 209 (Referring to FIG 9), and the swing crank 209 is fixedly connected to the swing connecting rod 211 through a crank pull pin 210. When the swing screw 205 moves back and forth, it drives the swing crank 209 to move back and forth, thereby driving the swing connecting rod 211 to move back and forth, so as to realize the left and right swing function of the distal actuator.

The electric stapler also includes a first bracket 105, a second bracket 105, a gear rack 203, the second bracket 200 and the gear rack 203 are fixed on the first bracket 105, the main drive assembly is fixed on the first bracket 105, the rotating motor 201 and the swing motor 202 are fixed on the second bracket 200, the gear assemblies of the rotating assembly and the swing assembly are arranged in the gear rack 203.

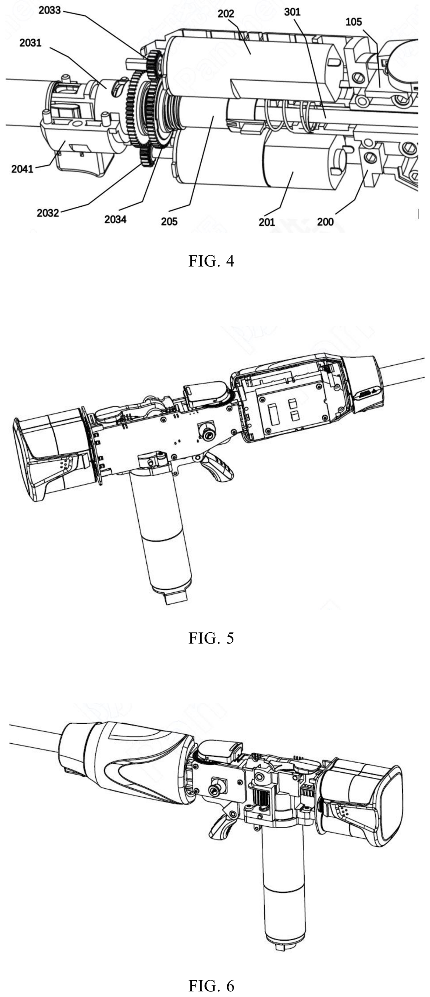

Further, referring to FIGs 5-6, the electric stapler includes a main circuit board 106 and a sub-circuit board 107, the main circuit board 106 and the sub-circuit board 107 are respectively fixed on both sides of the first bracket 105, the main circuit board 106 is provided with a control circuit and a first guide button 1061 of the electric stapler, and the sub-circuit board 107 is provided with a second guide button 1071, wherein, the first guide button 1061 and the second guide button 1071 are symmetrically arranged and have the same functions, the first guide button 1061 and the second guide button 1071 have trigger functions in five directions, namely, left and right, front and back, and downward pressing, which respectively correspond to the rotation of the actuator, the left and right swing of the actuator, and the start-firing confirmation function of the actuator.

After the stapler assembly is loaded, before closing, the angle and orientation of the actuator can be adjusted through the guide button according to surgical requirements. Pressing the center position of the guide button starts the firing confirmation function, but this function can only be used normally when the closure is in place. Each time the guide button is triggered by the operator in a direction, the on-off signal is transmitted to the main chip. After the main chip recognizes it, it controls the corresponding logic and cooperates with the actuator to realize the function. After the device is closed, it cannot adjust the angle and orientation of the cartridge assembly. If further adjustment is required, the device needs to be reset to its initial position.

Those skilled in the art will readily appreciate other embodiments of the present invention after considering the invention disclosed in the specification and examples. This application is intended to cover any variations, uses or adaptations of the present invention that follow the general principles of the present invention and include common knowledge or customary techniques in the art that are not disclosed in the present invention. The specification and examples are to be considered as exemplary only, and the true scope and spirit of the present invention are indicated by the claims.

It should be understood that the present invention is not limited to the exact construction that has been described above and shown in the drawings and that various modifications and changes may be made without departing from the scope thereof. The scope of the present invention is limited only by the appended claims.

## Claims

1. An electric stapler, which comprises a main drive assembly, a rotating assembly and a swing assembly;
wherein the main drive assembly is configured to drive an actuator to close, staple and retract,
the rotating assembly is configured to drive the actuator to rotate 360 degrees,
the swing assembly is configured to drive the actuator to swing left and right;
wherein each of the main drive assembly, the rotating assembly and the swing assembly comprises a motor and a gear assembly;
the main drive assembly, the rotating assembly and the swing assembly are able to be operated independently, without affecting each other during operations.

2. The electric stapler according to claim 1, wherein the main drive assembly includes: a firing motor, a firing gear, a firing driven gear, and a firing rack,
a distal end of the firing rack is connected to a proximal end of a firing push rod,
the firing push rod is able to rotate freely around the axis relative to the firing rack; the firing gear is engaged with the firing driven gear,
the firing driven gear is engaged with the firing rack,
when the firing motor rotates forward or reversely according to a main control instruction, a rotational motion is converted into a linear motion by the firing gear, the firing driven gear, and the firing rack, which drives a distal actuator to perform functions of closing, stapling and retracting .

3. The electric stapler according to claim 2, wherein the rotating assembly includes a rotating motor, a rotating drive gear, a rotating driven gear, the rotating assembly is connected to a barrel assembly through a fixed block,
wherein the rotating drive gear is engaged with the rotating driven gear,
the rotating driven gear includes a gear portion and a cylindrical portion,
the gear portion includes a center circular hole, and
one end of the cylindrical portion passes through the center circular hole and is fixed to the gear portion.

4. The electric stapler according to claim 3, wherein there are two fixed blocks, which are clamped together by a clamping assembly,
each fixed block is respectively provided with a first clamping platform and a second clamping platform,
the cylindrical portion is provided with a cylindrical portion clamping groove corresponding to the second clamping platform,
the fixed block is fixed to the rotating driven gear through the second clamping platform and the cylindrical portion clamping groove,
the fixed block is fixed to the barrel assembly through the first clamping platform and the barrel assembly clamping groove;
when the rotating driven gear rotates, the barrel assembly is driven to rotate through the fixed block, thereby realizing a rotation function of the distal actuator.

5. The electric stapler according to claim 4, wherein the swing assembly includes a swing motor, a swing screw, a swing drive gear and a swing driven gear,
the swing drive gear and the swing driven gear are engaged,
the swing driven gear and the swing screw are spirally matched,
the swing motor rotates to drive the swing screw to move back and forth through the swing drive gear and the swing driven gear;
the swing screw passes through the cylindrical portion of the rotating driven gear and is able to move back and forth in the cylindrical portion;
the swing screw is a hollow structure, and
the firing push rod passes through the swing screw and is able to move back and forth in the swing screw.

6. The electric stapler according to claim 5, wherein the swing driven gear is provided with a center hole, a wall of the center hole is provided with threads, an outer wall of the swing screw is provided with corresponding threads.

7. The electric stapler according to claim 5, wherein the swing assembly also includes a swing crank and a swing connecting rod, and
there is a crank ring groove on the swing screw, which cooperates with an annular clamping platform on the swing crank, and
the swing crank is fixedly connected to the swing connecting rod through a crank pull pin;
when the swing screw moves back and forth, it drives the swing crank to move back and forth, thereby driving the swing connecting rod to move back and forth, so as to realize the left and right swing function of the distal actuator.

8. The electric stapler according to claim 5, wherein the electric stapler also includes a first bracket, a second bracket, and a gear rack,
the second bracket and the gear rack are fixed on the first bracket,
the main drive assembly is fixed on the first bracket,
the rotating motor and the swing motor are fixed on the second bracket,
the gear assemblies of the rotating assembly and the swing assembly are arranged in the gear rack.

9. The electric stapler according to any one of claims 1 to 8, wherein the electric stapler also includes a main circuit board and a sub-circuit board,
the main circuit board and the sub-circuit board are respectively fixed on both sides of the first bracket, the main circuit board is provided with a control circuit and a first guide button of the electric stapler, and the sub-circuit board is provided with a second guide button,
wherein the first guide button and the second guide button are symmetrically arranged and have the same functions, and are used to control the operation of the stapler.

10. The electric stapler according to claim 9, wherein the first guide button and the second guide button have trigger functions in five directions: left, right, front, back, and downward pressing, which respectively correspond to the rotation of the actuator, the left and right swing of the actuator, and the start-firing confirmation of the actuator.
